(19) Europäisches Patentamt
European Patent Office
Office européen des brevets

(11) EP 0 839 795 B1

(12) **EUROPÄISCHE PATENTSCHRIFT**

(45) Veröffentlichungstag und Bekanntmachung des Hinweises auf die Patenterteilung:
**06.05.1999 Patentblatt 1999/18**

(51) Int. Cl.$^6$: **C07C 69/753**, C07C 67/10, C08F 218/04

(21) Anmeldenummer: **97118875.0**

(22) Anmeldetag: **30.10.1997**

(54) **Bicyclo- 2.2.2. -2.3:5.6-dibenzo-octadien-(2.5)-7-carbonsäure-alkenylester-Monomere und Copolymerisate dieser Alkenylester**

Alkenylester monomers of bicyclo-2,2,2-2,3:5,6-dibenzo-octadien-(2,5)-7-carboxylic acid and copolymers of these alkenyl esters

Monomères d'un ester d'alcényle de l'acide bicyclo-2,2,2-2,3:5,6-dibenzo-octadiène-(2,5)-7-carboxylique et copolymerisat de ces esters d'alcényle

(84) Benannte Vertragsstaaten:
**AT BE DE FR GB IT SE**

(30) Priorität: **31.10.1996 DE 19645026**

(43) Veröffentlichungstag der Anmeldung:
**06.05.1998 Patentblatt 1998/19**

(73) Patentinhaber:
**Consortium für Elektrochemische Industrie GmbH**
**81379 München (DE)**

(72) Erfinder:
- **Krueger, Benno, Dr.**
  **81369 München (DE)**
- **Huber, Ingrid**
  **81541 München (DE)**

(74) Vertreter:
**Schuderer, Michael, Dr. et al**
**Wacker-Chemie GmbH**
**Zentralabteilung Patente**
**Marken und Lizenzen**
**Hanns-Seidel-Platz 4**
**81737 München (DE)**

(56) Entgegenhaltungen:
- **Keine einschlägigen Dokumente gefunden**

EP 0 839 795 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

## Beschreibung

Die Erfindung betrifft Bicyclo-[2.2.2]-2.3:5.6-dibenzo-octadien-(2.5)-7-carbonsäure-alkenylester-Monomere sowie Copolymerisate dieser Alkenylester.

Die physikalischen Eigenschaften eines Polymers ändern sich oberhalb der Glastemperatur signifikant. Das Polymer verliert seine Steifheit und beginnt zu fließen. Die Glastemperaturen von Homopolymerisaten sind z. B. in J. Brandrup, E. H. Immergut, Polymer Handbook 1st Ed., J. Wiley, New York, 1966 und 2nd Ed., J. Wiley, New York 1975 aufgeführt. Für Copolymerisate kann die näherungsweise Berechnung der Glastemperaturen nach der Beziehung von Fox erfolgen. Nach Fox (T. G. Fox, Bull. Am. Phys. Soc. (Ser. II) 1, 123 [1956]) gilt für die Glastemperatur von Mischpolymerisaten in guter Näherung: $1/T_g = X_1/T_{g1} + X_2/T_{g2} + \ldots + X_n/T_{gn}$, wobei $X_1$, $X_2$, Xn die Massenbrüche der Monomeren 1, 2, ..., n und $T_{g1}$, $T_{g2}$, $T_{gn}$ die Glastemperaturen der jeweils nur aus einem der Monomeren 1,2,n aufgebauten Polymeren in Kelvin bedeuten.

Zur Erhöhung der Glastemperatur von Polymerisaten mit relativ niederer $T_g$ wird daher so vorgegangen, daß Monomere copolymerisiert werden, deren Homopolymerisate eine höhere $T_g$ aufweisen. Es ist bekannt, die Glastemperatur von Vinylester-Polymerisaten wie Vinylacetat-Polymeren (32°C) und Vinylacetat-Ethylen-Copolymeren (0°C bei ca. 15 % Ethylen-Anteil), oder die Glastemperatur von Acrylsäureester-Polymerisaten wie Butylacrylat-Polymeren (-54°C) durch Copolymerisation mit "harten" Monomeren, das heißt Monomeren deren Homopolymere eine höhere $T_g$ haben als die zu modifizierenden Polymerisate, zu erhöhen. Im Stand der Technik für diesen Zweck etablierte Comonomere sind beispielsweise Methylmethacrylat (105°C), Vinylchlorid (81°C) oder auch Versaticsäure-Vinylester wie VeoVa9® (60°C).

Die zuletzt genannten Standardcomonomere zur Erhöhung der Glastemperatur $T_g$ weisen jedoch eine Reihe von Nachteilen auf: Bei Methylmethacrylat handelt es sich um ein relativ teures Comonomer, welches mit Vinylestern nur sehr schwer copolymerisierbar ist. Nachteilig bei der Verwendung von Vinylchlorid als Comonomer zur Erhöhung des $T_g$-Wertes ist der Gehalt an Chlor. Vinylester von alpha-verzweigten Carbonsäuren mit 9 C-Atomen (VeoVa9®) haben den Nachteil der Verseifungsanfälligkeit bei Anwendung deren Copolymerisate im Alkalischen.

Es bestand daher die Aufgabe, ein hydrophobes Monomer zur Verfügung zu stellen, welches preiswert, technisch leicht herstellbar und schwer verseifbar ist, zum Einsatz in radikalischen Polymerisationen, insbesondere in Emulsionscopolymerisationen mit Vinylacetat, geeignet ist, und dessen Homopolymer eine so hohe $T_g$ aufweist, daß auch mit geringen Mengen eine deutliche Erhöhung des $T_g$-Wertes in einem Copolymerisat erhalten wird.

Gegenstand der Erfindung sind Alkenylester von Bicyclo-[2.2.2]-2.3:5.6-dibenzo-octadien-(2.5)-7-carbonsäuren mit der allgemeinen Formel

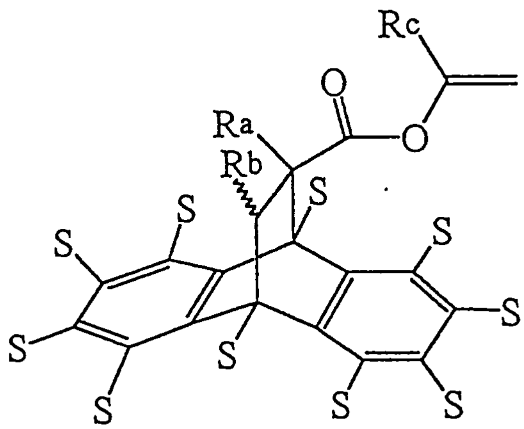

(I)

wobei $R^a$ und $R^b$ gleich oder verschieden sind und die Bedeutung H oder R haben, wobei R einen verzweigten oder unverzweigten $C_1$- bis $C_8$-Alkyl- oder Cycloalkylrest darstellt, $R^c$ die Bedeutung H oder $C_1$- bis $C_4$-Alkylrest hat, und S gleich oder verschieden sein kann und die Bedeutung H, R, OR, COOR, COR hat, wobei R einen verzweigten oder unverzweigten $C_1$- bis $C_8$-Alkyl- oder Cycloalkylrest darstellt.

Vorzugsweise sind $R^a$, $R^b$ und $R^c$ gleich oder verschieden und haben die Bedeutung H oder Methylrest. Die Reste S sind vorzugsweise gleich oder verschieden mit der Bedeutung H oder $C_1$- bis $C_8$-Alkylrest. Besonders bevorzugt sind die Alkenylester mit $R^a$ = H oder Methylrest, $R^b$ = H, $R^c$ = H oder Methylrest, und S = H. Am meisten bevorzugt wird der Bicyclo-[2.2.2]-2.3:5.6-dibenzo-octadien-(2.5)-7-carbonsäure-vinylester ($R^a$ = H, $R^b$ = H, $R^c$ = H, S = H) und der 7-Methyl-bicyclo-[2.2.2]-2.3:5.6-dibenzo-octadien-(2.5)-7-carbonsäurevinylester ($R^a$ = $CH_3$, $R^b$ = H, $R^c$ = H, S = H).

Die Herstellung der Alkenylester von substituierten Bicyclo-[2.2.2]-2.3:5.6-di-benzo-octadien-(2.5)-7-carbonsäuren mit der allgemeinen Formel (I) kann durch Umvinylierung von Vinylverbindungen mit den entsprechenden bicyclischen Carbonsäuren oder durch direkte Vinylierung der entsprechenden bicyclischen Carbonsäuren mit Acetylen erfolgen. Verfahren zur Alkenylester-Herstellung durch Umvinylierung oder Direktvinylierung sind beispielsweise aus der EP-A 54158 und aus K.Weissermel und H.-J. Arpe, Industrielle organische Chemie, 2. Aufl., S. 221-223, Verlag Chemie, Weinheim, New York, 1978 bekannt.

Vorzugsweise erfolgt die Herstellung der erfindungsgemäßen Alkenylester-Monomere durch Umvinylierung von substituierten Bicyclo-[2.2.2]-2.3:5.6-dibenzo-octadien-(2.5)-7-carbonsäuren der allgemeinen Formel

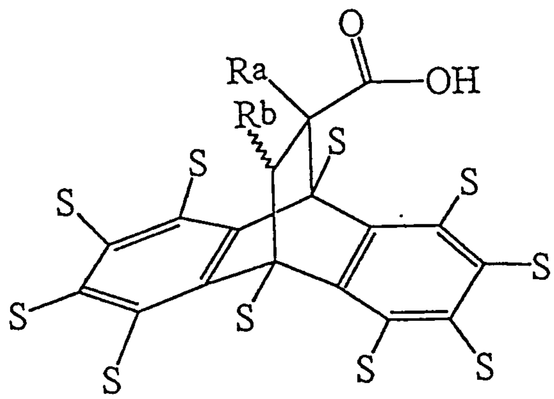

(II)

mit einer Alkenylverbindung, in Gegenwart von Stabilisatoren und Katalysatoren, wobei in der Formel (II) $R^a$ und $R^b$ gleich oder verschieden sind und die Bedeutung H oder R haben, wobei R einen verzweigten oder unverzweigten $C_1$- bis $C_8$-Alkyl- oder Cycloalkylrest darstellt, und S gleich oder verschieden sein kann und die Bedeutung H, R, OR, COOR, COR hat, wobei R einen verzweigten oder unverzweigten $C_1$- bis $C_8$-Alkyl- oder Cycloalkylrest darstellt.

Vorzugsweise sind $R^a$ und $R^b$ gleich oder verschieden und haben die Bedeutung H oder Methylrest. Die Reste S sind vorzugsweise gleich oder verschieden mit der Bedeutung H oder $C_1$- bis $C_8$-Alkylrest. Besonders bevorzugt sind die Carbonsäuren mit $R^a$ = H oder Methylrest, $R^b$ = H und S = H; das sind Bicyclo-[2.2.2]-2.3:5.6-dibenzo-octadien-(2.5)-7-carbonsäure ($R^a$ = H, $R^b$ = H, S = H) und 7-Methyl-bicyclo-[2.2.2]-2.3:5.6-dibenzo-octadien-(2.5)-7-carbonsäure ($R^a$ = $CH_3$, $R^b$ = H, S = H).

Geeignete Alkenylverbindungen zur Umvinylierung sind Vinylester von $C_1$- bis $C_8$-Alkylcarbonsäuren wie Vinylacetat, Vinylpropionat, sowie Propenylacetat, Isopropenylacetat und Vinylchlorid. Vorzugsweise wird Vinylacetat eingesetzt.

Die als Edukte eingesetzten Carbonsäuren der allgemeinen Formel (II) sind in an sich bekannter Weise durch Umsetzung von Anthracen oder entsprechend substituierten Anthracenderivaten in einer Diels-Alder-Reaktion mit ungesättigten Carbonsäuren wie Acryl- oder Methacrylsäure in Gegenwart eines Polymerisationsinhibitors erhältlich. Entsprechende Synthesewege sind beispielsweise in J. March, Advanced organic chemistry, 3rd Ed., p. 744-749, J. Wiley & Sons, New York, 1985 beschrieben.

Bei der Herstellung der Monomeren der allgemeinen Formel (I) werden die Alkenylverbindung und die bicyclische Carbonsäure der allgemeinen Formel (II) in einem molaren Verhältnis von 1 : 2 bis 1 : 80 eingesetzt. Wird die Umsetzung ohne Lösungsmittel durchgeführt, beträgt das molare Verhältnis von Alkenylverbindung zur bicyclischen Carbonsäure (II) vorzugsweise 1 : 5 bis 1 : 50; in Gegenwart von Lösungsmittel beträgt das molare Verhältnis vorzugsweise 1 : 2 bis 1 : 50. Bei kontinuierlicher Fahrweise beträgt das Verhältnis vorzugsweise 1 : 50 bis 1 : 100.

Die Reaktion wird unter Normaldruck, gegebenenfalls unter leichtem Überdruck, durchgeführt, falls die Reaktionstemperatur oberhalb der Siedetemperatur einer der im Reaktionsgemisch vorhandenen Komponenten liegt. Die Reaktionstemperatur beträgt im allgemeinen 50°C bis 150°C, vorzugsweise 50°C bis 120°C. Die Reaktionszeit beträgt, je nach Reaktionstemperatur, im allgemeinen 4 bis 80 Stunden, vorzugsweise 10 bis 50 Stunden. Gegebenenfalls kann auch unter Schutzgas, beispielsweise Stickstoff, zur Vermeidung oxidativer oder hydrolytischer Nebenreaktionen gearbeitet werden.

Im allgemeinen wird zur Umvinylierung die bicyclische Carbonsäure (II) in der Alkenylverbindung suspendiert oder gelöst. Die Reaktion wird vorzugsweise ohne Zusatz von Lösungsmittel durchgeführt. Es kann aber auch in Gegenwart von unter den Reaktionsbedingungen inerten Lösungsmitteln gearbeitet werden, beispielsweise Kohlenwasserstoffe wie Toluol oder Petrolether. Bei Einsatz von Lösungsmitteln beträgt das Gewichtsverhältnis von Lösungsmittel zu bicyclischer Carbonsäure (II) 1 : 1 bis 15 : 1.

Die Umsetzung wird in Gegenwart von Übergangsmetall-Katalysatoren aus der Gruppe der Hg(II)-Salze bzw. -Komplexe und der Platinmetallverbindungen wie Salze und Komplexe von Pt, Pd, Ru durchgeführt. Geeignete Katalysatoren sind beispielsweise die Acetate oder Halogenide von Hg(II), Pd(II) und Pt(II). Bevorzugt werden Komplexe von Pd-Acetat mit zweizähnigen Stickstoffliganden wie 2,2'-Bipyridin und 1,10-Phenantrolin. Die Katalysatoren werden in einem Molverhältnis Katalysator/bicyclische Carbonsäure (II) von 1/1000 bis 1/10 eingesetzt. Gegebenenfalls kann auch in Gegenwart von Cokatalysatoren aus der Gruppe der Mineralsäuren, Lewissäuren, Salze organischer oder anorganischer Säuren gemäß der Verfahrensweise aus der EP-A 54158 gearbeitet werden. Beispiele hierfür sind Schwefelsäure, Bor-Trihalogenide, Natriumacetat und Natriumchlorid. Der Cokatalysator wird dabei in einem Molverhältnis Katalysator/Cokatalysator von 1/1000 bis 1/2 eingesetzt. Die Katalysatoren und Cokatalysatoren können als solche oder auf Trägermaterialien aufgetragen eingesetzt werden. Geeignete Trägermaterialien sind beispielsweise Aktivkohle, Kieselgur.

Besonders bevorzugte Katalysatorsysteme sind folgende Kombinationen: Hg-II-Acetat und $H_2SO_4$, Hg-II-Acetat und $BF_3$-Etherat, Palladium-II-Chlorid/Natriumchlorid auf Aktivkohleträger, Palladium-II-Chlorid/Natriumchlorid auf Aktivkohleträger und Natriumacetat.

Bei der Durchführung des Verfahrens wird üblicherweise die umzusetzende Bicyclo-[2.2.2]-2.3:5.6-dibenzo-octadien-(2.5)-7-carbonsäure der allgemeinen Formel (II), gegebenenfalls in Anwesenheit eines Lösemittels in der Alkenylverbindung, vorzugsweise Vinylacetat oder Isopropenylacetat, suspendiert bzw. gelöst. Anschließend wird der Katalysator und gegebenenfalls der Cokatalysator zugegeben.

Alternativ ist bei festen oder trägergebundenen Katalysatorsystemen eine Reaktionsführung dergestalt möglich, daß der Katalysator dem Reaktionsgemisch nicht direkt zugegeben wird, sondern nur bei der Reaktionstemperatur mit diesem in Kontakt gebracht wird. Dies kann beispielsweise durch permanentes Pumpen der Reaktionslösung durch mit Katalysator gefüllte Reaktionsgefäße oder -röhren geschehen.

In einer bevorzugten Variante der Umsetzungsreaktion kann zur Verschiebung des Reaktionsgleichgewichtes, gegebenfalls nach Zugabe eines inerten höhersiedenden Lösemittels, - insbesondere bei festen Katalysatorsystemen - der Katalysator mittels einer Trennvorrichtung abgetrennt werden und danach Vinylacetat und entstandene Essigsäure abdestilliert werden und das Zwischenprodukt in einem zweiten Umvinylierungszyklus weiter umgesetzt werden. Dieser Vorgang kann mehrfach wiederholt werden.

Die Aufarbeitung des Ansatzes erfolgt nach dem für organische Feststoffe bekannten Stand der Technik, vorzugsweise mittels Aodestillation der nicht umgesetzten Alkenylverbindung und deren Folgeprodukt, im Fall von Vinylacetat des Vinylacetates, der entstandenen Essigsäure und leichtflüchtiger Verunreinigungen sowie Rückführung des Vinylacetates in den Prozeß.

In einer Variante des Verfahrens (bei in der Kälte im Reaktionsmedium schwerlöslichen Produkten) wird die Neutralisierung des Reaktionsansatzes sowie die Entfernung unlöslicher Bestandteile vor der Abdestillation, gegebenenfalls nach Zugabe eines inerten Lösemittels, durchgeführt.

In einer anderen Variante des Verfahrens, bei Verwendung fester Katalysatorsysteme oder bei auf Trägermaterialien aufgezogenen Katalysatorsystemen, kann der Katalysator vor der Destillation, beispielsweise durch Abpumpen der flüssigen Phase oder Abfiltration mittels einer Druckfilterpresse, entfernt werden. Überschüssige Alkenylverbindung sowie die entstandene Essigsäure werden danach, gegebenenfalls im Vakuum und unter Zugabe eines inerten Lösemittels, aodestilliert.

Die Isolierung und Aufreinigung der erfindungsgemäßen Bicyclo-[2.2.2]-2.3:5.6-dibenzo-octadien-(2.5)-7-carbonsäure-alkenylester der allgemeinen Formel (I) kann, wie aus dem Stand der Technik für organische Feststoffe bekannt, durch Umkristallisation aus inerten Lösemitteln, Fällung durch organische Lösungsmittel, Lösemittelextraktion, Aktivkohlebehandlung, Säulenchromatographie erfolgen.

Die erfindungsgemäßen Bicyclo-[2.2.2]-2.3:5.6-dibenzo-octadien-(2.5)-7-carbonsäure-alkenylester der allgemeinen Formel (I) eignen sich hervorragend als Comonomere in radikalischen Polymerisationsreaktionen in Fällen, in denen eine Erhöhung der Glasübergangstemperatur der Copolymerisationsprodukte zur Veränderung korrespondierender thermischer oder mechanischer Eigenschaften des Materials erwünscht ist.

Ein weiterer Gegenstand der Erfindung sind daher Copolymerisate der Bicyclo-[2.2.2]-2.3:5.6-dibenzooctadien-(2.5)-7-carbonsäure-alkenylester der allgemeinen Formel (I) mit einer oder mehreren copolymerisierbaren, ethylenisch ungesättigten Verbindungen. Bevorzugte ethylenisch ungesättigte Verbindungen sind (Meth)acrylsäureester von Alkoholen mit 1 bis 10 C-Atomen, Vinylester von gesättigten aliphatischen Carbonsäuren mit 2 bis 10 C-Atomen, Olefine, Vinylaromaten, Vinylhalogenide und/oder Vinylether.

Bevorzugt werden Copolymerisate mit 0.5 bis 30 Gew.-% an Comonomereinheiten der Bicyclo-[2.2.2]-2.3:5.6-dibenzo-octadien-(2.5)-7-carbonsäure-alkenylester der allgemeinen Formel (I) und Comonomereinheiten eines oder mehrerer Monomere aus der Gruppe der (Meth)acrylsäureester von Alkoholen mit 1 bis 10 C-Atomen, Vinylester von gesättigten aliphatischen Carbonsäuren mit 2 bis 10 C-Atomen, Olefine, Vinylaromaten, Vinylhalogenide und/oder Vinylether, welche gegebenenfalls noch weitere funktionelle Comonomereinheiten, beispielsweise vernetzbare Comonomere wie N-Methylol(meth)acrylamid enthalten. Die Angaben in Gew.-% beziehen sich jeweils auf das Gesamtgewicht des Copolymerisats. Vorzugsweise beträgt der Gehalt an Comonomereinheiten der bicyclischen Vinylester der allgemeinen Formel (I) 2 bis 20 Gew.-%.

Bevorzugte Basismonomere sind aus der Gruppe der Methacrylsäureester oder Acrylsäureester von Alkoholen mit 1 bis 10 C-Atomen Methylmethacrylat, Methylacrylat, Ethylmethacrylat, Ethylacrylat, Isopropylmethacrylat, Isopropylacrylat, tert.-Butylacrylat, n-Butylacrylat und Ethylhexylacrylat; aus der Gruppe der Vinylester von gesättigten aliphatischen Carbonsäuren mit 2 bis 10 C-Atomen Vinylacetat, Isopropenylacetat, Vinylpropionat, Vinyllaurat und Versaticsäure$^R$-Vinylester mit 9 bis 10 C-Atomen (Vinylester von gesättigten alpha-verzweigten Monocarbonsäuren, Handelsprodukt der Fa. Shell); aus der Gruppe der Olefine Ethylen, Propylen und 1,3-Butadien; aus der Gruppe der Vinylhalogenide Vinylchlorid und Styrol als bevorzugter Vinylaromat.

Gegebenenfalls können die erfindungsgemäßen Copolymerisate als Comonomere noch bis zu 10 Gew.-%, bezogen auf das Copolymerisat, ethylenisch ungesättigte, funktionelle Comonomere enthalten. Beispiele hierfür sind Mono- oder Dicarbonsäuren wie Methacrylsäure, Acrylsäure oder Fumarsäure und deren Amide, hydroxyfunktionelle Monomere wie Hydroxyethylacrylat, 2-Hydroxypropylacrylat oder N-Methylolacrylamid, sulfonatfunktionelle Monomere wie Vinylsulfonat oder 2-Acrylamido-2-methylpropansulfonat und mehrfach ungesättigte Monomere wie Divinyladipat.

Besonders bevorzugt sind Copolymerisate von einem oder mehreren Comonomeren aus der Gruppe Vinylacetat, Isopropenylacetat, Vinylpropionat, Vinyllaurat, Vinylchlorid, 1.3-Butadien und/oder Ethylen und Bicyclo-[2.2.2]-2.3:5.6-dibenzo-octadien-(2.5)-7-carbonsäure-vinylester und/oder 7-Methyl-bicyclo-[2.2.2]-2.3:5.6-dibenzo-octadien-(2.5)-7-carbonsäurevinylester.

Am meisten bevorzugt sind Copolymerisate mit Ethylen und/oder Acrylsäureester wie n-Butylacrylat und Ethylhexylacrylat, und/oder Vinylacetat und/oder Vinylchlorid und 2 bis 20 Gew.-% Bicyclo-[2.2.2]-2.3:5.6-dibenzo-octadien-(2.5)-7-carbonsäure-vinylester und/oder 7-Methyl-bicyclo-[2.2.2]-2.3:5.6-dibenzo-octadien-(2.5)-7-carbonsäurevinylester.

Die Copolymerisate der bicyclischen Vinylester der allgemeinen Formel (I) können durch Polymerisation in Masse, in Lösung, in Suspension oder in Emulsion hergestellt werden. Von den genannten Verfahren ist die Emulsionspolymerisation die bevorzugte Variante. Die Polymerisation kann diskontinuierlich oder kontinuierlich, mit oder ohne Verwendung von Saatlatices, unter Vorlage aller oder einzelner Bestandteile des Reaktionsgemisches, oder unter teilweiser Vorlage und Nachdosierung der oder einzelner Bestandteile des Reaktionsgemisches, oder nach dem Dosierverfahren ohne Vorlage, durchgeführt werden. Alle Dosierungen erfolgen vorzugsweise im Maße des Verbrauchs der jeweiligen Komponente.

Der Start der Polymerisationsreaktion erfolgt vorzugsweise durch radikalische Initiierung. Um bei niedrigen Temperaturen initiieren zu können, kann die Initiierung auch in Redox-Fahrweise durchgeführt werden. Bei der bevorzugten Emulsionspolymerisation erfolgt die Initiierung vorzugsweise mittels geeigneter wasserlöslicher Radikalbildner, die vorzugsweise in Mengen von 0.01 bis 3.0 Gew.-%, bezogen auf das Gesamtgewicht der Monomeren, eingesetzt werden. Beispiele hierfür sind Ammonium- und Kaliumpersulfat oder Wasserstoffperoxid. Als Reduktionsmittel eignen sich die in solchen Polymerisationsverfahren üblichen Systeme, wie sie in Houben-Weyl Band 14/1, Seiten 263-297, aufgeführt sind, beispielsweise Natriumformaldehydsulfoxylat, Natriumsulfit, Natriumhydrogensulfit, Dithionit oder Ascorbinsäure.

Als Dispergiermittel können alle üblicherweise bei der Emulsionspolymerisation verwendeten Emulgatoren und Schutzkolloide eingesetzt werden. Vorzugsweise werden 1 bis 6 Gew.-%, bezogen auf das Gesamtgewicht der Monomeren, an Emulgator eingesetzt. Geeignet sind beispielsweise anionische Tenside, wie Alkylsulfate mit einer Kettenlänge von 8 bis 18 C-Atomen, Alkyl- und Alkylarylethersulfate mit 8 bis 18 C-Atomen im hydrophoben Rest und bis zu 40 Ethylen- oder Propylenoxideinheiten, Alkyl- oder Alkylarylsulfonate mit 8 bis 18 C-Atomen, Ester und Halbester der Sulfobernsteinsäure mit einwertigen Alkoholen oder Alkylphenolen. Geeignete nichtionische Tenside sind beispielsweise Alkylpolyglykolether oder Alkylarylpolyglykolether mit 8 bis 40 Ethylenoxideinheiten.

Gegebenenfalls können Schutzkolloide, vorzugsweise in Mengen bis zu 15 Gew.-%, bezogen auf das Gesamtgewicht der Monomeren, eingesetzt werden. Beispiele hierfür sind Vinylalkohol/Vinylacetat-Copolymere mit einem Gehalt von 80 bis 100 Mol% Vinylalkoholeinheiten, Polyvinylpyrrolidone mit einem Molekulargewicht von 5000 bis 400000, Hydroxyethylcellulosen mit einem Substitutionsgradbereich von 1.5 bis 3.

Der für die Polymerisation gewünschte pH-Bereich, der im allgemeinen zwischen 2.5 und 10, vorzugsweise 3 und 8, liegt, kann in bekannter Weise durch Säuren, Basen oder übliche Puffersalze, wie Alkaliphosphate oder Alkalicarbonate, eingestellt werden. Zur Molekulargewichtseinstellung können bei der Polymerisation die üblicherweise verwendeten Regler, zum Beispiel Mercaptane, Aldehyde und Chlorkohlenwasserstoffe, zugesetzt werden.

Die Vorteile der erfindungsgemäßen Bicyclo-[2.2.2]-2.3:5.6-dibenzo-octadien-(2.5)-7-carbonsäure-alkenylester der allgemeinen Formel (I) lassen sich wie folgt zusammenfassen:

Es handelt sich um hydrophobe Monomere, welche in den gängigen Solventien der radikalischen Lösungspolymerisation sowie in gängigen Monomeren wie Vinylacetat gut löslich sind. Durch ihre Hydrophobie sind die erfindungsmäßigen Monomeren auch sehr gut zur Durchführung von Emulsionspolymerisationen geeignet.

Die erfindungsgemäßen Verbindungen der Formel (I) sind sehr "harte" Monomere, deren Homopolymerisate eine extrem hohe Glastemperatur $T_g$ oberhalb der Zersetzungstemperatur des Homopolymers haben. Daraus folgt ein erheblicher ökonomischer Vorteil, da zur Erhöhung der Glasübergangstemperatur von Copolymeren nur geringe Mengen der erfindungsgemäßen Monomeren in ein Copolymer eingebaut werden müssen. Ferner tritt durch diese geringen Mengen nur eine relativ geringe Änderung der Grundeigenschaften des Polymers auf.

Die erfindungsmäßigen Monomeren sind aufgrund der Halogenfreiheit ökologisch von Vorteil und können als Ersatzstoff für chlorhaltige Monomere wie Vinylchlorid benutzt werden, die zur Zeit noch in großen Mengen zur Erhöhung der Glastemperatur von Copolymeren eingesetzt werden.

Schließlich liegt ein weiterer ökonomischer Vorteil darin, daß die erfindungsgemäßen Monomere technisch leicht und preiswert herstellbar sind und in einer großen Bandbreite bezüglich der unterschiedlichen Polymerisationstypen und Comonomere einsetzbar sind.

Hervorzuheben ist auch die hohe Verseifungsstabilität der Copolymeren: Bei den erfindungsgemäßen bicyclischen Vinylestermonomeren sind im Polymeren die bicyclischen Reste als Ester tertiärer Carbonsäuren an der Hauptkette fixiert, woraus eine hohe Verseifungsstabilität in stark basischen Medien wie Beton, Putzen etc. resultiert. Entsprechende Copolymere sind daher besonders gut für Anwendungen im Baubereich geeignet. Generell eignen sich die Copolymere als Bindemittel in Beschichtungen und Klebemittel.

Die nachfolgenden Beispiele dienen zur weiteren Erläuterung der Erfindung:

a) Synthese der Edukte:

Beispiel 1:

Bicyclo-[2.2.2]-2.3:5.6-dibenzo-octadien-(2.5)-7-carbonsäure.

534 g Anthracen und 324 g Acrylsäure wurden in 1.26 l Xylol insgesamt 15 h am Rückfluß erhitzt, wobei nach 9 h und 13 h jeweils 100 ml bzw. 20 ml Acrylsäure nachgegeben wurden. Nach dem Abkühlen wurde das Rohprodukt abgesaugt und mit Wasser gewaschen. Der getrocknete Feststoff wurde aus Ethylacetat umkristallisiert. Die Ausbeute betrug 529 g.

Beispiel 2:

7-Methyl-bicyclo-[2.2.2]-2.3:5.6-dibenzo-octadien-(2.5)-7-carbonsäure.

356 g Anthracen und 258 g Methacrylsäure wurden in 840 l Xylol insgesamt 16 h am Rückfluß erhitzt, wobei nach 8 h weitere 100 g Methacrylsäure nachgegeben wurden. Nach dem Abkühlen wurde das Rohprodukt abgesaugt und mit Wasser gewaschen. Der getrocknete Feststoff wurde aus Ethylacetat umkristallisiert. Die Ausbeute betrug 363 g.

b) Synthese der erfindungsgemäßen Alkenylester:

Beispiel 3:

Bicyclo-[2.2.2]-2.3:5.6-dibenzo-octadien-(2.5)-7-carbonsäurevinylester.

Unter Stickstoffschutzgas wurden 64.0 g Bicyclo-[2.2.2]-2.3:5.6-dibenzo-octadien-(2.5)-7-carbonsäure in 540 ml Vinylacetat unter Erwärmen gelöst, 0.98 g Quecksilber-II-acetat zugegeben und 0.5 h gerührt. Nach Zugabe von 96 µl konz. Schwefelsäure wurde 5 h am Rückfluß gerührt, 0.51 g Natriumacetat zugegeben und das erkaltete Reaktionsgemisch durch Filtration von unlöslichen Bestandteilen befreit. Überschüssiges Vinylacetat sowie sonstige flüchtige Verbindungen wurden im Vakuum abgezogen und das erhaltene Rohprodukt aus Isopropanol umkristallisiert. Ausbeute: 48.5 g, Schmelzpunkt: 74°C.

Beispiel 4:

7-Methyl-bicyclo-[2.2.2]-2.3:5.6-dibenzo-octadien-(2.5)-7-carbonsäurevinylester.

Unter Stickstoffschutzgas wurden 100 g 7-Methyl-bicyclo-[2.2.2]-2.3:5.6-dibenzo-octadien-(2.5)-7-carbonsäure in 744 ml Vinylacetat unter Erwärmen gelöst, 1.53 g Quecksilber-II-acetat zugegeben und 0.5 h gerührt. Nach Zugabe von 140 µl konz. Schwefelsäure wurde 13 h am Rückfluß gerührt, 0.79 g Natriumacetat zugegeben und das erkaltete Reaktionsgemisch durch Filtration von unlöslichen Bestandteilen befreit. Überschüssiges Vinylacetat sowie sonstige flüchtige Verbindungen wurden im Vakuum abgezogen und das erhaltene Rohprodukt aus Isopropanol umkristallisiert. Ausbeute: 86.4 g, Schmelzpunkt:91°C.

Beispiel 5:

7-Methyl-bicyclo-[2.2.2]-2.3:5.6-dibenzo-octadien-(2.5)-7-carbonsäurevinylester.

9.42 g 7-Methyl-bicyclo-[2.2.2]-2.3:5.6-dibenzo-octadien-(2.5)-7-carbonsäure, 90 ml Vinylacetat und 1.08 g Palladiumchlorid / Natriumchlorid Katalysator auf Aktivkohle (3 %) wurden in Gegenwart von 2.5 mg Benzochinon 36 h am Rückfluß gerührt und danach die erkaltete Lösung filtriert. Das Filtrat wurde dreimal mit je 20 ml Wasser und danach mit 20 ml gesättigter Natriumhydrogencarbonatlösung gewaschen. Nach Trocknung über Natriumsulfat wurden bei 50°C im Vakuum flüchtige Bestandteile abgezogen und danach das Produkt im Vakuum getrocknet. Ausbeute: 8.40 g.

Beispiel 6:

7-Methyl-bicyclo-[2.2.2]-2.3:5.6-dibenzo-octadien-(2.5)-7-carbonsäurevinylester.

28.6 g 7-Methyl-bicyclo-[2.2.2]-2.3:5.6-dibenzo-octadien-(2.5)-7-carbonsäure, 270 ml Vinylacetat und 1.62 g Palladiumchlorid / Natriumchlorid Katalysator auf Aktivkohle (3 %) wurden in Gegenwart von 7.5 mg Benzochinon 24 h am Rückfluß gerührt. Der gaschromatographisch bestimmte Umsatz des Eduktes zum Vinylester lag bei 57.3 %. Bei Normaldruck wurde aus dem Ansatz das Vinylacetat und danach i. Vak. die Essigsäure abdestilliert. Der Rückstand wurde abgekühlt, 270 ml Vinylacetat zugegeben und weitere 42 h am Rückfluß gerührt. Der gaschromatographisch bestimmte Umsatz des Eduktes zum Vinylester lag bei 93.6 %.

Beispiel 7:

7-Methyl-bicyclo-[2.2.2]-2.3:5.6-dibenzo-octadien-(2.5)-7-carbonsäurevinylester.

9.42 g 7-Methyl-bicyclo-[2.2.2]-2.3:5.6-dibenzo-octadien-(2.5)-7-carbonsäure, 45 ml Vinylacetat und 0.81 g Palladiumchlorid / Natriumchlorid Katalysator auf Aktivkohle (3 %) wurden in Gegenwart von 2.5 mg Benzochinon 30 h am Rückfluß gerührt. Der gaschromatographisch bestimmte Umsatz des Eduktes zum Vinylester lag bei 98.5 %.

Beispiel 8:

7-Methyl-bicyclo-[2.2.2]-2.3:5.6-dibenzo-octadien-(2.5)-7-carbonsäurevinylester.

9.42 g 7-Methyl-bicyclo-[2.2.2]-2.3:5.6-dibenzo-octadien-(2.5)-7-carbonsäure, 90 ml Vinylacetat und 0.81 g Palladiumchlorid / Natriumchlorid Katalysator auf Aktivkohle (3 %) wurden in Gegenwart von 2.5 mg Benzochinon 7 h bei 120°C im Autoklaven gerührt. Der gaschromatographisch bestimmte Umsatz des Eduktes zum Vinylester lag bei 82.0 %.

c) Polymerisationen:

Beispiel 9:

Lösungscopolymerisation von Bicyclo-[2.2.2]-2.3:5.6-dibenzo-octadien-(2.5)-7-carbonsäurevinylester mit Vinylacetat und Bestimmung der Glasübergangstemperaturen.

Zu einer Lösung von Vinylacetat (VAc) und unterschiedlichen Anteilen der jeweiligen Bicyclo-[2.2.2]-2.3:5.6-dibenzo-octadien-(2.5)-7-carbonsäurevinylester (VE) in Methanol (MeOH) wurde unter Rühren nach Aufheizen zum Rückfluß Dilauroylperoxid (DLPO) zugegeben. Die Ansätze wurden jeweils mindestens 3 h am Rückfluß gerührt. Nach Ausreaktion der Monomeren wurde das Methanol bei erhöhter Badtemperatur weitgehend abdestilliert und danach der Polymerrückstand im Vakuum von restlichem Lösungsmittel und Monomeren befreit. Das Polymer wurde in Tetrahydrofuran gelöst, aus Ethanol umgefällt und im Vakuumtrockenschrank von restlichem Lösemittel befreit. Die mittels DSC-Analytik bestimmten Glasübergangstemperaturen der erhaltenen Copolymeren sowie die genauen Einsatzmengen der Edukte sind in Tabelle 1 wiedergegeben.

Tabelle 1

| Versuch Nr. | VAc [g] | MeOH [g] | DLPO [g] | VE [g] | ($R^b$) | $T_g$ [°C] |
|---|---|---|---|---|---|---|
| 1 | 51.6 | 50.0 | 0.67 | 2.5 | (H) | 48.6 |
| 2 | 50.0 | 50.0 | 0.67 | 5.0 | (H) | 52.8 |
| 3 | 100.0 | 100.0 | 1.33 | 5.0 | (Me) | 46.8 |
| 4 | 50.0 | 50.0 | 0.67 | 5.0 | (Me) | 49.6 |
| 5 | 100.0 | 100.0 | 1.33 | 20.0 | (Me) | 54.7 |

Vergleichsbeispiel 1:

Emulsionspolymerisation von Vinylacetat-Homopolymer

Die Dispersionen wurden in einem 21-Reaktionsgefäß mit Ankerrührer hergestellt. Die Monomeren und Hilfsstoffe wurden mittels Feindosiergefäßen zugegeben. Die Katalysatoren (APS/Brüggolit) wurden als 4 %-ige bzw. 2 %-ige Lösungen mit einer Schlauchpumpe eindosiert. Die Temperatur wurde mittels einer Thermostatisiereinrichtung und eines Innentemperaturreglers bei 45°C konstant gehalten.
Vorlage: 387 g Wasser, 0.83 g Sulfobernsteinsäurehalbester von Monofettalkoholen mit 10 bis 12 C-Atomen (30 %-ige Lösung) , 7.7 g Ethylenoxid-Propylenoxid-Copolymerisat (20 %-ige Lösung), 2.0 g Vinylsulfonat als 25 %-ige Lösung, 2.7 g Benzochinon als 0.1 %-ige Lösung, 48.8 g Vinylacetat, 2.0 g Ferroammonsulfat als 1 %-ige Lösung.
Die Vorlage wurde mit 10 %-iger Ameisensäure auf pH 3.8 eingestellt und die Dosierungen 1 und 2 zugeführt:

Dosierung 1 (Dosierzeit ca. 6 h): 3.00 g Ammoniumperoxodisulfat in 60 ml Wasser.
Dosierung 2 (Dosierzeit ca. 6 h): 1.50 g Natriumhydroxymethansulfinat in 60 ml Wasser.
Nach Anspringen der Polymerisation wurde mit den Dosierungen 3 und 4 begonnen:
Dosierung 3 (Dosierzeit ca. 6 h): 468.8 g Vinylacetat.
Dosierung 4 (Dosierzeit ca. 6 h): 40.0 g Wasser, 10.3 g sulfatiertes Alkylethoxylat mit ca. 3 EO-Einheiten (28-%-ige Lösung), 36.0 g Isotridecylethoxylat mit 15 EO-Einheiten (40 %-ige Lösung), 31.3 g Vinylsulfonat als 25 %-ige Lösung.

Es wurde eine Dispersion mit einem Feststoffgehalt von 44.6 Gew%. und einem K-Wert von 62.7 erhalten. Die $T_g$ des Copolymeren wurde mittels DSC-Analytik ermittelt und betrug 37.9°C.

Beispiel 10:

Emulsionscopolymerisation von 7-Methyl-bicyclo-[2.2.2]-2.3:5.6-dibenzo-octadien-(2.5)-7-carbonsäurevinylester mit Vinylacetat.

Die Dispersionen wurden in einem 2l-Reaktionsgefäß mit Ankerrührer hergestellt. Die Monomeren und Hilfsstoffe wurden mittels Feindosiergefäßen zugegeben. Die Katalysatoren (APS/Brüggolit) wurden als 4 %-ige bzw. 2 %-ige Lösungen mit einer Schlauchpumpe eindosiert. Die Temperatur wurde mittels einer Thermostatisiereinrichtung und eines Innentemperaturreglers bei 45°C konstant gehalten.
Vorlage: 387 g Wasser, 0.83 g Sulfobernsteinsäurehalbester von Monofettalkoholen mit 10 bis 12 C-Atomen (30 %-ige Lösung), 7.7 g Ethylenoxid-Propylenoxid-Copolymerisat (20 %-ige Lösung), 2.0 g Vinylsulfonat als 25 %-ige Lösung, 3.0 g Benzochinon als 0.1 %-ige Lösung, 48.8 g Vinylacetat, 2.0 g Ferroammonsulfat als 1 %-ige Lösung.
Die Vorlage wurde mit 10 %-iger Ameisensäure auf pH 3.8 eingestellt und die Dosierungen 1 und 2 zugeführt:

Dosierung 1 (Dosierzeit ca. 6 h): 3.00 g Ammoniumperoxodisulfat in 60 ml Wasser.
Dosierung 2 (Dosierzeit ca. 6 h): 1.50 g Natriumhydroxymethansulfinat in 60 ml Wasser.
Nach Anspringen der Polymerisation wurde mit den Dosierungen 3 und 4 begonnen:
Dosierung 3 (Dosierzeit ca. 6 h): 445.3 g Vinylacetat und 26.3 g 7-Methyl-bicyclo-[2.2.2]-2.3:5.6-dibenzo-octadien-(2.5)-7-carbonsäurevinylester.
Dosierung 4 (Dosierzeit ca. 6 h): 40.0 g Wasser, 10.3 g sulfatiertes Alkylethoxylat mit ca. 3 EO-Einheiten (28-%-ige Lösung), 36.0 g Isotridecylethoxylat mit 15 EO-Einheiten (40 %-ige Lösung), 31.3 g Vinylsulfonat als 25 %-ige

Lösung.

Es wurde eine Dispersion mit einem Feststoffgehalt von 42.5 Gew%. und einem K-Wert von 63.8 erhalten. Die $T_g$ des Copolymeren wurde mittels DSC-Analytik ermittelt und betrug 42°C.

## Patentansprüche

1. Bicyclo-[2.2.2]-2.3:5.6-dibenzo-octadien-(2.5)-7-carbonsäure-alkenylester-Monomer mit der allgemeinen Formel

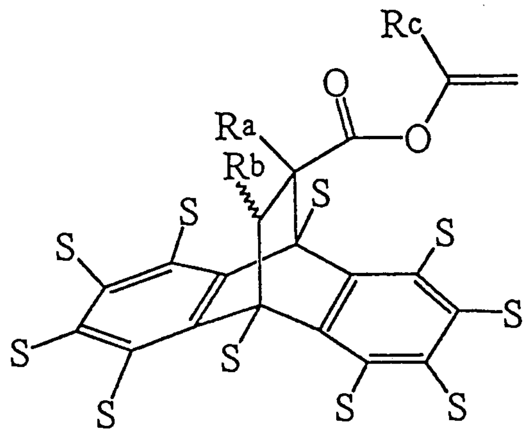

(I)

wobei $R^a$ und $R^b$ gleich oder verschieden sind und die Bedeutung H oder R haben, wobei R einen verzweigten oder unverzweigten $C_1$- bis $C_8$-Alkyl- oder Cycloalkylrest darstellt, $R^c$ die Bedeutung H oder $C_1$- bis $C_4$-Alkylrest hat, und S gleich oder verschieden sein kann und die Bedeutung H, R, OR, COOR, COR hat, wobei R einen verzweigten oder unverzweigten $C_1$- bis $C_8$-Alkyl- oder Cycloalkylrest darstellt.

2. Alkenylester-Monomer nach Anspruch 1, dadurch gekennzeichnet, daß $R^a$ = H oder Methylrest, $R^b$ = H, $R^c$ = H oder Methylrest, und S = H.

3. Bicyclo-[2.2.2]-2.3:5.6-dibenzo-octadien-(2.5)-7-carbonsäure-vinylester ($R^a$ = H, $R^b$ = H, $R^c$ = H, S = H) und 7-Methylbicyclo-[2.2.2]-2.3:5.6-dibenzo-octadien-(2.5)-7-carbonsäurevinylester ($R^a$ = $CH_3$, $R^b$ = H, $R^c$ = H, S = H).

4. Verfahren zur Herstellung der Alkenylester-Monomere gemäß Anspruch 1, durch Umvinylierung von substituierten Bicyclo-[2.2.2]-2.3:5.6-dibenzo-octadien-(2.5)-7-carbonsäuren der allgemeinen Formel

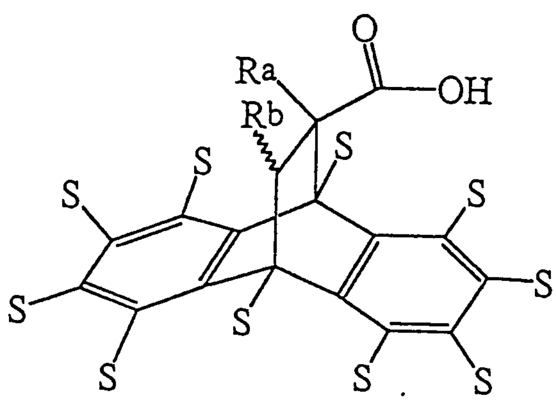

(II)

mit Vinylestern von $C_1$- bis $C_8$-Alkylcarbonsäuren oder Vinylchlorid, in Gegenwart von Stabilisatoren und Katalysatoren, wobei in der Formel (II) $R^a$ und $R^b$ gleich oder verschieden sind und die Bedeutung H oder R haben, wobei R einen verzweigten oder unverzweigten $C_1$- bis $C_8$-Alkyl- oder Cycloalkylrest darstellt, und S gleich oder verschieden sein kann und die Bedeutung H, R, OR, COOR, COR hat, wobei R einen verzweigten oder unverzweigten $C_1$-

bis $C_8$-Alkyl- oder Cycloalkylrest darstellt.

5. Copolymerisate von Bicyclo-[2.2.2]-2.3:5.6-dibenzooctadien-(2.5)-7-carbonsäure-vinylester nach Anspruch 1 bis 3 mit einer oder mehreren copolymerisierbaren ethylenisch ungesättigten Verbindungen.

6. Copolymerisate nach Anspruch 5, mit 0.5 bis 30 Gew.-% an Comonomereinheiten der Bicyclo-[2.2.2]-2.3:5.6-dibenzo-octadien-(2.5)-7-carbon-säure-vinylester nach Anspruch 1 bis 3 und Comonomereinheiten eines oder mehrerer Monomere aus der Gruppe der (Meth)acrylsäureester von Alkoholen mit 1 bis 10 C-Atomen, Vinylester von gesättigten aliphatischen Carbonsäuren mit 2 bis 10 C-Atomen, Olefine, Vinylaromaten, Vinylhalogenide und/oder Vinylether, welche gegebenenfalls noch weitere funktionelle Comonomereinheiten enthalten.

7. Copolymerisate gemäß Anspruch 5 und 6, von einem oder mehreren Comonomeren aus der Gruppe Vinylacetat, Isopropenylacetat, Vinylpropionat, Vinyllaurat, Vinylchlorid, Ethylen und Bicyclo-[2.2.2]-2.3:5.6-dibenzo-octadien-(2.5)-7-carbonsäurevinylester und/oder 7-Methyl-bicyclo-[2.2.2]-2.3:5.6-dibenzo-octadien-(2.5)-7-carbonsäurevinylester.

8. Verfahren zur Herstellung von Copolymerisaten gemäß Anspruch 5 bis 7, durch Polymerisation in Masse, in Lösung, in Suspension oder in Emulsion.

## Claims

1. Bicyclo-[2.2.2]-2.3:5.6-dibenzo-octadiene-(2.5)-7-carboxylic acid alkenyl ester monomer of the general formula

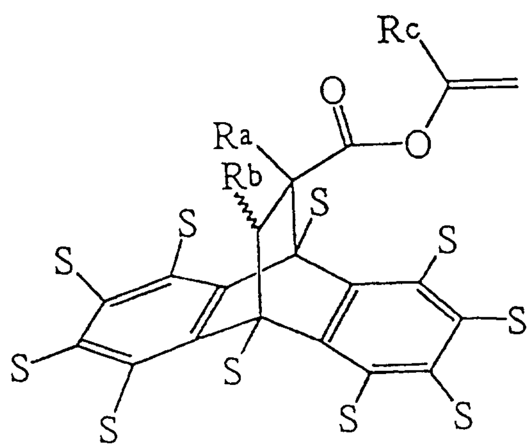

(I)

in which $R^a$ and $R^b$ are identical or different and are H or R, where R is a branched or unbranched $C_1$- to $C_8$-alkyl or a cycloalkyl radical, $R^c$ is H or a $C_1$- to $C_4$-alkyl radical, and S can be identical or different and is H, R, OR, COOR or COR, where R is a branched or unbranched $C_1$- to $C_8$-alkyl or a cycloalkyl radical.

2. Alkenyl ester monomer according to Claim 1, characterized in that $R^a$ = H or a methyl radical, $R^b$ = H, $R^c$ = H or a methyl radical and S = H.

3. Bicyclo-[2.2.2]-2.3:5.6-dibenzo-octadiene-(2.5)-7-carboxylic acid vinyl ester ($R^a$ = H, $R^b$ = H, $R^c$ = H, S = H) and 7-methyl-bicyclo-[2.2.2]-2.3:5.6-dibenzo-octadiene-(2.5)-7-carboxylic acid vinyl ester ($R^a$ $CH_3$, $R^b$ = H, $R^c$ = H, S = H).

4. Process for the preparation of the alkenyl ester monomers according to Claim 1, by transvinylation of substituted bicyclo-[2.2.2]-2.3:5.6-dibenzooctadiene-(2.5)-7-carboxylic acids of the general formula

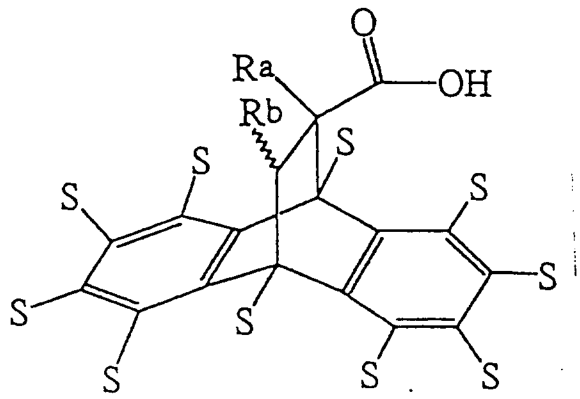

(II)

with vinyl esters of $C_1$- to $C_8$-alkyl carboxylic acids or vinyl chloride in the presence of stabilizers and catalysts, in which, in the formula (II), $R^a$ and $R^b$ are identical or different and are H or R, where R is a branched or unbranched $C_1$- to $C_8$-alkyl or a cycloalkyl radical, and S can be identical or different and is H, R, OR, COOR or COR, where R is a branched or unbranched $C_1$- to $C_8$-alkyl or a cycloalkyl radical.

5. Copolymers of bicyclo-[2.2.2]-2.3:5.6-dibenzooctadiene-(2.5)-7-carboxylic acid vinyl ester according to Claim 1 to 3 with one or more copolymerizable ethylenically unsaturated compounds.

6. Copolymer according to Claim 5, having 0.5 to 30% by weight of comonomer units of bicyclo-[2.2.2]-2.3:5.6-dibenzo-octadiene-(2.5)-7-carboxylic acid vinyl esters according to Claim 1 to 3 and comonomer units of one or more monomers from the group consisting of (meth)acrylic acid esters of alcohols having 1 to 10 C atoms, vinyl esters of saturated aliphatic carboxylic acids having 2 to 10 C atoms, olefins, vinyl aromatics, vinyl halides and/or vinyl ethers, which optionally also comprise further functional comonomer units.

7. Copolymers according to Claim 5 and 6, of one or more comonomers from the group consisting of vinyl acetate, isopropenyl acetate, vinyl propionate, vinyl laurate, vinyl chloride, ethylene and bicyclo-[2.2.2]-2.3:5.6-dibenzo-octadiene-(2.5)-7-carboxylic acid vinyl ester and/or 7-methyl-bicyclo-[2.2.2]-2.3:5.6-dibenzooctadiene-(2.5)-7-carboxylic acid vinyl ester.

8. Process for the preparation of copolymers according to Claim 5 to 7, by polymerization in bulk, in solution, in suspension or in emulsion.

**Revendications**

1. Monomère ester alcénylique de l'acide bicyclo-[2,2,2]-2,3:5,6-dibenzo-octadiène-(2,5)-7-carboxylique de formule générale

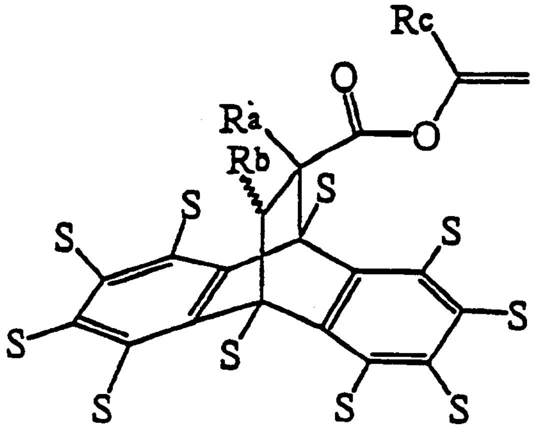

(I)

dans laquelle $R^a$ et $R^b$ sont identiques ou différents et sont H ou R, où R est un radical alkyle en $C_1$ à $C_8$ ramifié ou non ramifié ou un radical cycloalkyle, $R^c$ est H ou un radical en $C_1$ à $C_4$, et S peut être identique ou différent et est H, R, OR, COOR, COR, où R représente un radical alkyle en $C_1$ à $C_8$ ramifié ou non ramifié ou un radical cycloalkyle.

**2.** Monomère ester alcénylique selon la revendication 1, caractérisé en ce que $R^a$ = H ou un radical méthyle, $R^b$ = H, $R^c$ = H ou un radical méthyle et S = H.

**3.** Ester vinylique de l'acide bicyclo-[2,2,2]-2,3:5,6-dibenzo-octadiène-(2,5)-7-carboxylique ($R^a$ = H, $R^b$ = H, $R^c$ = H, S = H) et ester vinylique de l'acide 7-méthyl-bicyclo-[2,2,2]-2,3:5,6-dibenzo-octadiène-(2,5)- 7-carboxylique ($R^a$ = $CH_3$, $R^b$ = H, $R^c$ = H, S = H).

**4.** Procédé de préparation des monomères ester alcénylique selon la revendication 1, par transvinylation d'acides bicyclo-[2,2,2]-2,3:5,6-dibenzo-octadiène-(2,5)-7-carboxyliques substitués de formule générale

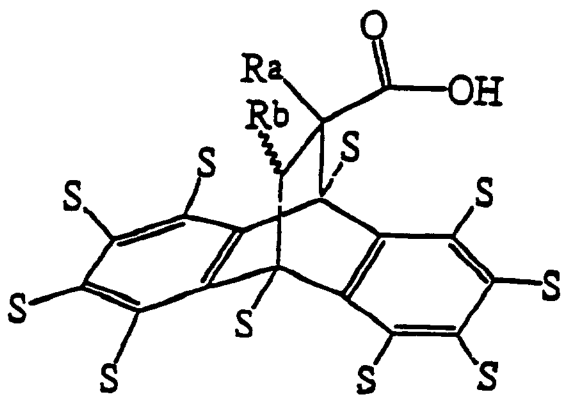

(II)

avec des esters vinyliques d'acides $C_1$-$C_8$-alkylcarboxyliques ou du chlorure de vinyle, en présence de stabilisants et de catalyseurs, où dans la formule (II), $R^a$ et $R^b$ sont identiques ou différents et sont H ou R, où R est un radical alkyle en $C_1$ à $C_8$ ramifié ou non ramifié ou un radical cycloalkyle, et S peut être identique ou différent et est H, R, OR, COOR, COR, où R représente un radical alkyle en $C_1$ à $C_8$ ramifié ou non ramifié ou un radical cycloalkyle.

**5.** Copolymères d'esters vinyliques de l'acide bicyclo-[2,2,2]-2,3:5,6-dibenzo-octadiène-(2,5)-7-carboxylique selon les revendications 1 à 3, avec un ou plusieurs composés copolymérisables éthyléniquement insaturés.

**6.** Copolymères selon la revendication 5, ayant de 0,5 à 30% en poids de motifs co-monomères d'esters vinyliques de l'acide bicyclo-[2,2,2]-2,3:5,6-dibenzooctadiène-(2,5)-7-carboxylique selon les revendications 1 à 3 et des

motifs co-monomères d'un ou de plusieurs monomères parmi le groupe constitué d'esters (méth)acryliques d'alcools ayant de 1 à 10 atomes de carbone, d'esters vinyliques d'acides carboxyliques aliphatiques saturés ayant de 2 à 10 atomes de carbone, d'oléfines, de composés vinyle aromatiques, d'halogénures de vinyle et/ou d'éthers vinyliques, qui renferment éventuellement également d'autres motifs comonomères fonctionnels.

**7.** Copolymères selon les revendications 5 et 6, d'un ou de plusieurs co-monomères parmi le groupe constitué de l'acétate de vinyle, de l'acétate d'isopropényle, du propionate de vinyle, du laurate de vinyle, du chlorure de vinyle, de l'éthylène et d'esters vinyliques de l'acide bicyclo-[2,2,2]-2,3:5,6-dibenzo-octadiène-(2,5)-7-carboxylique et/ou d'esters vinyliques de l'acide 7-méthyl-bicyclo-[2,2,2]-2,3:5,6-dibenzo-octadiène-(2,5)-7-carboxylique.

**8.** Procédé de préparation de copolymères selon les revendications 5 à 7, par polymérisation en masse, en solution, en suspension ou en émulsion.